(19) Europäisches Patentamt
European Patent Office
Office européen des brevets

(11) EP 2 805 721 B1

(12) **EUROPEAN PATENT SPECIFICATION**

(45) Date of publication and mention
of the grant of the patent:
**05.09.2018 Bulletin 2018/36**

(51) Int Cl.:
*A61K 31/7004* (2006.01)  *A61K 36/185* (2006.01)
*A61K 36/48* (2006.01)  *A61K 36/54* (2006.01)
*A61K 36/60* (2006.01)  *A61P 25/00* (2006.01)

(21) Application number: **13168825.1**

(22) Date of filing: **23.05.2013**

(54) **Mimicking the metabolic effect of caloric restrictions by administration of glucose anti-metabolites to enhance positive response in a mammal**

Nachahmung der Stoffwechselauswirkungen der kalorischen Einschränkung durch Verabreichung von Glucoseantimetaboliten zur Verbesserung der positiven Reaktion bei einem Säugetier

Imiter l'action métabolique de restrictions caloriques par administration d'anti-métabolites de glucose pour améliorer la réponse positive chez un mammifère

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.11.2014 Bulletin 2014/48**

(73) Proprietor: **IAMS Europe B.V.**
**7742 PB Coevorden (NL)**

(72) Inventors:
• **Davenport, Gary Mitchell**
**Mason, OH Ohio 45040 (US)**
• **Shoveller, Anna Katharine**
**Mason, OH Ohio 45040 (US)**
• **Gooding, Margaret Ann**
**Lewisburgh, OH Ohio 45338 (US)**
• **Ingram, Donald Keith**
**Baton Rouge, LA Louisiana 70809 (US)**

(74) Representative: **Zimmermann & Partner**
**Patentanwälte mbB**
**Postfach 330 920**
**80069 München (DE)**

(56) References cited:
**WO-A1-2011/069051  US-A1- 2002 035 071**

• **Y. YAMAMOTO ET AL: "Changes in behavior and gene expression induced by caloric restriction in C57BL/6 mice", PHYSIOLOGICAL GENOMICS, vol. 39, no. 3, 8 September 2009 (2009-09-08), pages 227-235, XP055075293, ISSN: 1094-8341, DOI: 10.1152/physiolgenomics.00082.2009**
• **ROWLAND ET AL: "Physiological and behavioral responses to glucoprivation in the golden hamster", PHYSIOLOGY AND BEHAVIOR, ELSEVIER SCIENCE LTD., OXFORD, GB, vol. 30, no. 5, 1 May 1983 (1983-05-01), pages 743-747, XP024533710, ISSN: 0031-9384, DOI: 10.1016/0031-9384(83)90172-5 [retrieved on 1983-05-01]**

**Description**

FIELD OF THE INVENTION

The present invention relates to the use of glucose anti-metabolites to alter utilization of glucose or other carbohydrate sources and to mimic metabolic effects of caloric restriction for the purpose of enhancing positive response in a mammal.

BACKGROUND OF THE INVENTION

One of the most vigorous techniques to promote optimal glucose and insulin profiles is calorie restriction (CR) or calorie restriction mimetics (CRM). CRMs have been studied as an alternative to CR and to avoid some of the negative effects of CR regimens. The objectives of CRM strategies are to produce the same pro-longevity effects that CR provides without reducing caloric intake. Since the pro-longevity strategies of CR influence systems involved in energy sensing, and regulation of metabolism, some targets of CRMs focused on metabolites that modify glucose metabolism, one of the primary pathways used for the energy production via storage or catabolism.

Glucose anti-metabolites such as 2-deoxy-D-glucose are compounds related to glucose. However, due to structural differences from glucose such compounds block or inhibit certain aspects of carbohydrate metabolism and may therefore mimic the effects of caloric restriction. These glucose anti-metabolites exert a number of physiological effects; including reduction of body weight, decrease in plasma insulin levels, reduction of body temperature, retardation of tumor formation and growth, and elevation of circulating glucocorticoid hormone concentrations. These physiological effects result from inhibition of carbohydrate metabolism. The present invention relates to effect of inhibition of carbohydrate metabolism to cause positive affect and positive arousal.

The terms affect and arousal have a long history of use in psychology with many possible interpretations. Both are hypothetical constructs used to describe specific aspects of behavior.

The affect and the mood are connected and need to be understood together. As a hypothetical construct, *mood* is an internal, subjective state driven by feelings that can be expressed verbally in humans. Two types of moods are generally described: positive mood or negative mood. Negative mood is associated with feelings of depression, poor self esteem, aggression, anxiety, stress and irritability; whereas, positive mood is associated with feelings of energy and increased motivation, alertness, sense of well-being, friendliness, and trustworthiness. However, the mood can be inferred from observable behavioral responses in humans and nonhuman mammals.

To address this distinction, another hypothetical construct is applied, *affect,* which refers to the description of an individual's externally displayed mood based on behavioral responses. Thus, while we are apt to describe mood in nonhuman mammals, such as companion animals; technically we are inferring their mood based upon affective responses. Thus, the term affect is a more suitable description of the psychological state according to the present invention. In addition, the term, *affect display,* can be applied as this is a conventional psychological term referring to the facial, vocal, gestural or postural behavior that indicates affect. The affect is considered to comprise both positive and negative dimensions. The positive affect scale reflects the level of pleasant engagement, reflecting the extent to which a person or animal feels enthusiastic, excited, active, and determined; whereas, the negative affect scale reflects a general dimension of unpleasant engagement and subjective distress that subsumes a broad range of aversive affects including fear, nervousness, guilt, and shame. The present invention relates to behavioral indications of the positive affect.

In the psychology literature, arousal is a hypothetical construct that refers to a physiological state of being awake and reacting to stimuli, a process leading to increased sensory alertness, mobility, and readiness to respond. In effect, arousal is involved in regulating consciousness, attention, and information processing. Based on context of the stimuli presented and motivational drives engaged, the resulting behaviors in an aroused state can be either mobilizing or immobilizing. Another important aspect of the hypothetical construct of arousal is that of the curvilinear relationship to performance. Known as the Yerkes-Dodson Law, this view emphasizes that there is an optimal level of arousal for performance such that too little or too much arousal can adversely affect task performance. At a neural level, a state of arousal is driven by the reticular activating system in the brain and the autonomic nervous system and endocrine system in the body. Physiological soliloquy can include increased heart rate and blood pressure. Additionally, hormonal responses, such increased levels of plasma glucocorticoids and free fatty acids have been suggested as measures of arousal. Arousal can also be inferred physiologically by brain activity measured by electroencephalography (EEG). Thus, it is important to understand that the present invention relates to an increase in arousal that do not adversely affect performance but rather move the treated mammal toward optimized performance. Moreover, in considering effects of the present invention on affect and arousal, we can define positive affect as a measure of energetic arousal; whereas, negative affect relates to feelings of unpleasant arousal.

Notable behavioral responses have also been reported in animals and humans undergoing calorie restriction which are indicative of positive affect and increased arousal. Specifically, when rodents are fed calorie restricted diets, they demonstrate increased locomotor activity in general and also in exploration of novel objects and areas. This can be interpreted as a natural response designed to increase food-seeking behavior. In bar-pressing tasks, rodents fed short-term calorie restricted diets demonstrate greater amounts of spontaneous bar presses and also increased willingness to bar-press for food rewards. These observations would meet the descriptions of positive affect including being more active, focused, determined, attentive, inspired, and alert. These behaviors are also indicative of an increased state of energetic arousal. Regarding physiological indicators of arousal, rodents on short-term calorie restriction show increased heart rate and blood pressure, but those on long-term calorie restriction for several weeks show reduced heart rate and blood pressure. However, biochemical indicators of arousal, such as glucocorticoids and free fatty acids, are elevated in rodents on short-term calorie restriction and this elevation persists over long periods.

The lateral hypothalamus, referred to as the "feeding center" is responsive to plasma glucose levels. Increased glucose oxidation and electrical activity of the lateral hypothalamus and a brief transient decline in plasma glucose all precede feeding in humans and rats and are correlated to feelings of hunger. The induction of a diabetogenic state with mannoheptulose (MH) or other glucose anti-metabolite consumption may reduce glucose responsiveness in the lateral hypothalamus, thus impacting satiety/hunger signals associated with glucose metabolism. Therefore, hunger, that affects the motivational system underlying play behavior in the domestic cat, is also influenced by glucose metabolism. Since glucose anti-metabolite such as MH inhibits glucose metabolism and reduces glucose responsiveness then perhaps the metabolic effect of MH would be indicated by observed increase in activity of the cat through enhanced demonstrations of play.

Behavior challenges in both familiar and unfamiliar situations are known to increase the physiological and psychological stress levels of mammals and to decrease quality of life. An unknown situation can be fear- and/or stress-inducing and may be more pronounced when mammals undergo change of exposure to a novel situation. This may lead the mammal to be negative, not fully engaged, less active, and not fully aware of the environment. When a mammal has a positive affect, it can help the mammal to perceive this as an opportunity to learn and investigate rather than responding as if in a fearful situation, in which the mammal may stop performing. Also an increase in arousal that do not adversely affect performance but rather move the treated mammal toward optimized performance will help in these familiar and unfamiliar situations.

US 2002/035071 relates to a method of obtaining beneficial biological results associated with caloric restriction that may be gained by administration of a composition containing at least one active agent which blocks metabolism of glucose as a source of energy in cells in glucose metabolism blocking effective amounts to an animal in need thereof.

In an article of Rowland et al., Physiology and Behavior 30, 5 (May 1983), the physiological and behavioral responses to glucoprivation in the golden hamster have been investigated, and it has been shown that golden hamsters failed to increase their food intake following food deprivation alone or in combination with insulin or 2-deoxy-D-glucose (2DG) treatment.

It is thus an objective of the present invention to provide a composition suitable for use by mammals in familiar and/or unfamiliar situations to enhance positive affect and/or energetic arousal.

It has been found that the above objective can be met by the use of glucose anti-metabolite in a composition according to the present invention.

It is an advantage of the compositions according to the present invention that they may be used to get mammal engaged in your daily life because he is happier and motivated to play.

It is another advantage of the compositions according to the present invention that they may be used to get mammal fully engaged, active, positive and fully aware of environment.

SUMMARY OF THE INVENTION

The present invention relates to a use of composition comprising a glucose anti-metabolite in a method of enhancing a positive effect and/or energetic arousal in a mammal as defined in the appended claims.

BRIEF DESCRIPTION OF THE DRAWINGS

Figure 1 is a graphical presentation of results relating to comfort in new situations, when handler and evaluator are familiar.
Figure 2 is a graphical presentation of results relating to comfort in new situations, when handler is familiar and evaluator is unfamiliar.
Figure 3 is a graphical presentation of results relating to comfort in new situations, when handler and evaluator are unfamiliar.
Figure 4 is a graphical presentation of results relating to social interactions, when handler and evaluator are familiar.

Figure 5 is a graphical presentation of results relating to social interaction, when handler is familiar and evaluator is unfamiliar.

Figure 6 is a graphical presentation of results relating to social interactions, when handler and evaluator are unfamiliar.

Figure 7 is a graphical presentation of old and young cohorts pooled together, the time by diet interaction showing serum Trp:LNAA.

Figure 8 is a graphical presentation of old and young cohorts separately, the time by diet interaction showing serum Trp:LNAA.

DETAILED DESCRIPTION OF THE INVENTION

All percentages and ratios are calculated by weight unless otherwise indicated. All percentages and ratios are calculated based on the total composition unless otherwise indicated.

Referenced herein are trade names for components including various ingredients utilized in the present invention. The inventors herein do not intend to be limited by materials under a certain trade name. Equivalent materials (*e.g.,* those obtained from a different source under a different name or reference number) to those referenced by trade name may be substituted and utilized in the descriptions herein.

In the description of the invention various embodiments or individual features are disclosed. As will be apparent to the ordinarily skilled practitioner, all combinations of such embodiments and features are possible and can result in preferred executions of the present invention.

The compositions herein may comprise, consist essentially of, or consist of any of the features or embodiments as described herein.

All oral doses of the invention are calculated per kilogram of body weight of the mammal unless otherwise indicated.

Humans and companion animals are advantageously treated herein. As used herein, "companion animal" means a domestic animal. Preferably, "companion animal" means a dog, cat, rabbit, ferret, horse, cow, or the like. More preferably, "companion animal" means a dog or cat.

The present invention is directed to use of compositions comprising a glucose anti-metabolite for use to enhance a positive effect and/or energetic arousal in a mammal. A positive affect and/or energetic arousal in said mammal is selected from the group consisting of enhanced social interaction, enhanced obedience, enhanced manners, enhanced alertness, enhanced awareness, enhanced activity and mixtures thereof.

Positive affect and/or positive arousal lead the mammal to be fully engaged, focused, active, alert and fully aware of the environment. Without intending to be limited by theory, it is believed that glucose availability for neurons enhances/maintains nerve function, additionally glucose sustains the energy levels for brains which leads mammal to be able to be fully functional for longer periods. Brain primarily utilizes glucose as its main energy source to synthesize cellular energy ATP. There is evidence that glucose levels fluctuate. Glucose anti-metabolite such as mannoheptulose may provide more sustained level of glucose and prevent glucose fluctuation, and therefore, provide sustained energy levels for brains.

Without intending to be limited by theory, it is believed that enhancement of the positive affect leads to increase of energetic arousal. By the increase in arousal is meant that arousal do not adversely affect performance but rather move the treated mammal towards optimized performance.

The present invention relates to the use of glucose anti-metabolite components to alter utilization of glucose or other carbohydrate sources and to mimic metabolic effects of caloric restriction.

The glucose anti-metabolites which are useful herein include 2-deoxy-D-glucose, 5-thio-D-glucose, 3-O-methylglucose, anhydrosugars including 1,5-anhydro-D-glucitol, 2,5-anhydro-D-glucitol, and 2,5-anhydro-D-mannitol, and mannoheptulose (MH). Mannoheptulose is most preferred glucose anti-metabolite for use herein. Without intending to be limited by theory, these compounds are accepted to be glucose anti-metabolites. Also without intending to be limited by theory, it is believed that mannoheptulose is glucose anti-metabolite. See e.g., U.S. patent application Publication No. 2002/0035701. Advantageously, mannoheptulose may be present in the recited compositions as a component of plant matter such as an avocado extract, avocado meal or other enriched source of mannoheptulose. Non-limiting examples of enriched sources of mannoheptulose are alfalfa, fig or primrose. The plant matter may include the fruit, seed (or pit), branches, leaves, or any other portion of the relevant plant or combinations thereof.

Avocado (also commonly referred to as alligator pear, aguacate, or palta) contains unusually enriched levels of mannoheptulose, as well as related sugars and other carbohydrates. Avocado is a sub-tropical evergreen tree fruit, growing most successfully in areas of California, Florida, Hawaii, Guatemala, Mexico, the West Indies, South Africa, and Asia.

Species of avocado include, for example, *Persea Americana* and *Persea Nubigena,* including all cultivars within these illustrative species. Examples of suitable cultivars may include Anaheim; Bacon; Creamhart; Duke; Fuerte; Ganter; Gwen; Hass; Jim; Lula; Lyon; Mexicola Grande; Murrieta Green; Nabal; Pinkerton; Queen; Puebla; Reed; Rincon; Ryan;

Spinks; Topa Topa; Whitsell; Wurtz; and Zutano. The fruit of the avocado is particularly preferred for use herein, which may contain the pit or wherein the pit is removed or at least partially removed. Fruit from *Persea Americana* is particularly preferred for use herein, as well as fruit from cultivars which produce larger fruits (e.g., about 12 ounces or more when the fruit is mature), such as Anaheim, Creamhart, Fuerte, Hass, Lula, Lyon, Murrieta Green, Nabal, Queen, Puebla, Reed, Ryan and Spinks.

A particularly preferred avocado is a criollo avocado. The criollo may be a member selected from the group consisting of criollo West Indian avocado, criollo West Indian/Guatemalan hybrid avocado and mixtures thereof, especially avocado grown in the Dominican Republic. Optimally, said criollo avocado is an early harvest criollo avocado.

Plant matter from alfalfa, fig, or primrose is also reported to provide relatively high levels of mannoheptulose. Alfalfa is also referred to as *Medicago sativa.* Fig, or *Ficus carica* (including Cluster fig or Sycamore fig, for example) may also be used, as well as primrose or *Primula officinalis.*

It has been discovered that particular levels of a glucose anti-metabolite selected from 2-deoxy-D-glucose; 5-thio-D-glucose; 3-O-methylglucose; 1,5-anhydro-D-glucitol; 2,5-anhydro-D-glucitol; 2,5-anhydro-D-mannitol; mannoheptulose; and mixtures thereof are useful herein. In particular, it has been found that relatively low levels, as well as relatively high doses of the glucose anti-metabolite, while useful, may provide less than optimal efficacy for desired purposes. Dosage will depend upon the glucose anti-metabolite used and will vary depending upon the size and condition of the mammal to which the glucose anti-metabolite is to be administered. Dosage of mannoheptulose, for example, in the range of 0.0005 to 1 g/kg, or from 0.001 to 1 g/kg is beneficial, g/kg meaning gram per kilogram of body weight of the mammal. Dosage at the lower range would be appropriate when using 2-deoxy-D-glucose in large animals. Higher dosage, particularly of compounds such as 5-thio-D-glucose or mannitol would be readily tolerated. In an embodiment, the dosage of the glucose anti-metabolite provided to a mammal on a daily basis may be from 0.5 to 200 mg/kg, preferably from 1 to 150 mg/kg more preferably from 2 to 100 mg/kg, wherein "mg" refers to the level of the component and "kg" refers to kilograms of body weight of the mammal. In an embodiment, the dosage of the mannoheptulose provided to a mammal on a daily basis may be from 0.5 to 20 mg/kg, preferably from 1.0 to 10mg/kg and more preferably from 2 to 8mg/kg. In certain embodiments, this may translate to compositions comprising less than 10% by weight of the composition of the glucose anti-metabolite, or less than 5%, or less than 2%, or from 0.0001% to 0.5% by weight of the composition of the anti-glucose metabolite. The level of glucose anti-metabolite may be determined by one of ordinary skill in the art based on a variety of factors, for example, the form of the composition (*e.g.,* whether a dry composition, semi-moist composition, wet composition, or supplement, or any other form or mixture thereof). The ordinarily skilled artisan will be able to utilize the preferred dosage and determine the optimal level of glucose anti-metabolite within a given composition.

Similarly, wherein an extract or meal of plant matter is utilized in the compositions herein, optimal levels of extract or meal may be dependent upon level of efficacious component within such extract or meal. Optimal extracts and/or meals have been found herein which comprise from 0.5% to 99% by weight of the extract or meal of the glucose anti-metabolite, alternatively from 0.5% to 75% of the glucose anti-metabolite component, alternatively from 0.5% to 50% of the glucose anti-metabolite component, alternatively, from 0.5% to 25% of the glucose anti-metabolite component. Optimal extracts and/or meals have been found herein in which glucose anti-metabolite may be from 0.5 to 99% by weight of the extract and/or meal, preferably from 1 to 75%, more preferably from 5 to 50% and most preferably from 10 to 25%.

The composition may comprise avocado flesh plus a member selected from avocado pit, avocado peel, or both pit and peel. The composition may comprise an aqueous extract of avocado comprising mannoheptulose or another glucose anti-metabolite.

The present invention is directed to a composition that is intended for ingestion by a mammal. Compositions include foods intended to supply necessary dietary requirements, as well as treats (*e.g.,* biscuits) or other food supplements. Optionally, the composition herein may be a dry composition (for example, kibble), semi-moist composition, wet composition, or any mixture thereof. Alternatively or additionally, the composition is a supplement, such as gravy, sauce, drinking water, yogurt, powder, beverage, suspension, chew, treat (*e.g.,* biscuits), supplemental water and combination thereof.

The composition is nutritionally balanced food or pet food. As used herein, the term "nutritionally balanced," with reference to the composition, means that the composition has known required nutrients to sustain life in proper amounts and proportion based on recommendations of recognized authorities in the field of nutrition. Most preferably the composition is dog or cat food.

The compositions used herein may optionally comprise one or more further components. Other components are beneficial for inclusion in the compositions used herein, but are optional for purposes of the invention. In one embodiment, the compositions may comprise, on a dry matter basis, from 10% to 90% by weight of the composition of crude protein, preferably from 20% to 50%, more preferably from 20% to 40%, and most preferably from 20% to 35% by weight of the composition of crude protein. The crude protein material may comprise vegetable-based proteins such as soybean, cereals (corn, wheat, etc), cottonseed, and peanut, or animal-based proteins such as casein, albumin, and meat protein. Non-limiting examples of meat protein useful herein include a protein source selected from the group consisting of beef, pork, lamb, poultry, fish, and mixtures thereof.

Furthermore, the compositions may comprise, on a dry matter basis, from 5% to 40% by weight of the composition of fat, more preferably from 10% to 35%.

The compositions of the invention may further comprise a source of carbohydrate. In one embodiment, the compositions may comprise from 35% up to 50 by weight of the composition of carbohydrate source. In other embodiments, the composition can comprise from 35% to 45%, by weight of the composition of carbohydrate source, preferably from 40% to 50%. Grains or cereals such as rice, corn, milo, sorghum, barley, wheat, and the like are illustrative sources of carbohydrate.

The compositions may also contain other materials such as, but not limited to, dried whey and other dairy by-products, beet pulp, cellulose, fiber, fish oil, flax, vitamins, minerals, flavors, and antioxidants.

The compositions may also contain other optional ingredients. Optional ingredients can include probiotic components (Bifidobacteria and/or Lactobacillus) and prebiotic (fructooligosaccharides) components. Examples and amounts of probiotic components and prebiotic components that can be included are disclosed in United States Publication No. 2005/0158294, for example. Other optional ingredients that can be included are omega-6 and omega-3 fatty acids, hexametaphosphate, glucosamine, chondroitin sulfate, carotenoids including beta carotene, vitamin E, and lutein, and those ingredients as shown in Table 3 below.

The following non-limiting illustrations exemplify the various glucose anti-metabolites of the present invention:

*Decreased Utilization of Glucose as Energy Source by 2-Deoxy-D-Glucose:*

To mimic the effects of caloric restriction, glucose anti-metabolites are provided over an extended time period. Previous studies show that 2-deoxy-D-glucose should not be administered in high doses, since significant untoward side effects and toxicity have been observed. However, studies in rodents (Lane et al., J. Anti-Aging Med. 1 (4): 327 - 337 (1998)) have shown that long-term disruption of glucose metabolism using a lower dose of 2-deoxy-D-glucose can mimic some of the major metabolic hallmarks of caloric restriction and enhanced longevity, including reduced body temperature, weight loss, and lower fasting insulin levels.

In light of the above potential physiologic benefits of caloric restriction weighed against the negative aspects of metabolic inhibition by 2-deoxy-D-glucose, alternatives which act as anti-metabolites of glucose without the potentially harmful side effects are preferred for purposes of practicing the invention.

*Decrease of Availability of Glucose to Cells by 5-Thio-D-Glucose:*

5-Thioglucose, an analog of glucose, has (*in vivo*) more pronounced effects than 2-deoxy-D-glucose. The compound is believed to act mainly by inhibiting glucose uptake by the cells. The majority of 5-thioglucose (97%) injected into a rat has been found excreted unchanged in urine (Hoffman et al., Biochemistry 7, pp. 4479 - 4483 (1968)). 5-Thioglucose is remarkably non-toxic; $LD_{50}$ was measured to be 14 g/kg, by injection, in rats (Chen et al., Arch. Biochem. Biophys., 169, pp. 392 - 396 (1975)).

Since 5-Thio-D-glucose, like mannoheptulose and other glucose anti-metabolites, seems to be excreted unchanged in urine, this compound and others present certain advantages for chronic administration over 2-deoxy-D-glucose. Since 5-thio-D-glucose and other glucose anti-metabolites inhibit glucose uptake, appropriate dosing can result in benefits associated with caloric restriction, including enhanced health, wellness and longevity.

*Effects of 3-O-Methylglucose:*

This analog of glucose, in contrast with 2-deoxy-D-glucose, is not metabolized (Jay et al., J. Neurochem. 55, pp. 989 - 1000 (1990)) and, thus, may provide certain advantages for use in chronic administration. In the context of this invention, 3-O-methylglucose can prevent utilization of glucose as an energy source as demonstrated by response to its administration in rats. The responses were about seven times weaker than those to 2-deoxyglucose.

*Effects of Anhydrosugars: 1,5-Anhydro-D-Glucitol (Polygalitrol):*

This compound is a non-reducing analog of glucose and is enzymatically converted to 1,5-anhydro-D-glucitol-6-phosphate, albeit the conversion is less efficient than that of 2-deoxy-glucose (Sols et al., J. Biol. Chem., 210, pp. 581 - 595 (1954). 1,5-anhydro-D-glucitol-6-phosphate is an allosteric (non-competitive) inhibitor of hexokinase, which catalyzes the first regulatory step of glycolysis (Crane et al., J. Biol. Chem., 210, pp. 597 - 696 (1954)). Furthermore, 1,5-anhydro-D-glucitol-6-phosphate is a non-reducing analog and cannot be a substrate for the next step of glycolysis catalyzed by glucose-6-phosphate isomerase. Consequently, this analog could accumulate in cells and act as a very effective metabolic block to glucose utilization. Another advantage relating to its non-reducing character is that this compound cannot be incorporated into glycolipids, glycoproteins, and glycogen. Thus, its effects are specific to glycolysis and would not be expected to affect other metabolic processes or exert toxicity of some glucose anti-metabolites previously discussed.

Interestingly, this compound (or its phosphate) has been found in the human body. It was found to be present in cerebrospinal fluid of patients who had occasional high blood glucose (from diabetes and diseases of the kidney) in large enough concentrations to be detected in tests performed in normal clinical settings.

*Use of 2,5-Anhydro-D-Mannitol and 2,5-Anhydro-D-Glucitol:*

These compounds are non-reducing analogs of fructose. Fructose is an important component of food and fructose phosphates and diphosphate are intermediate products of glycolysis. Nevertheless, inhibition of metabolic events involving fructose and its phosphates by anhydrosugar analogs is difficult. Alpha and beta anomers of fructose, which spontaneously inter-convert, correspond to different anhydrosugars, to 2,5-anhydro-D-glucitol and 2,5-anhydro-D-mannitol, respectively. Thus, only a few of the enzymatic conversions can be inhibited by a single compound. The 2,5-Anhydro-D-mannitol has been investigated in some detail. That compound is taken up by cells and converted into 2,5-anhydro-D-mannitol-1-phosphate. That phosphate is an analog of fructose-1-phosphate, but cannot be cleaved by the aldolase and, thus, the utilization of both glucose and fructose by cells is blocked. The 2,5-Anhydro-D-mannitol had been found to interfere in glucose formation and utilization in isolated rat hepatocytes (Riquelme et al., Proc. Natl. Acad. Sci. USA, 80, pp. 431-435 (1983)).

*Decrease of Glucose Utilization as Energy Source by Ketoses:*

Mannoheptulose (MH) is present in reasonable amounts in some foods (*e.g.,* avocados may contain up to 5% of mannoheptulose, by wet weight) and can be classified as a "generally recognized as safe" substance for human consumption. In studies of metabolism, doses of 10 grams of mannoheptulose were safely administered to humans orally. About 5% of the mannoheptulose ingested was reported to appear in urine after oral administration. The fate of injected mannoheptulose has previously been investigated in rats: 66% was excreted unchanged, 29% was metabolized and, a day after the injection, 5% remained in the body (Simon et al., Arch. Biochem. Biophys, 69, pp. 592-601 (1957)). Mannoheptulose is preferred glucose anti-metabolite due its high abundance in natural sources and due its safety profile.

The availability of glucose to cells can also be decreased using other dietary supplements than those specifically identified herein which have similar effect on metabolism of glucose that can result in an inhibition of glucose processing.

The methods of the invention may be practiced by administering a component described herein orally or parenterally, though oral administration would be preferred. When lowering of tissue metabolism is desired, as an adjunct to treatment of trauma, the component may be administered intravenously.

EXAMPLES

The following examples are provided to illustrate the invention and are not intended to limit the scope thereof in any manner.

Example 1

Calorie restriction mimetic (MH) effect on affective behavior

Annual kennel assessments (period of 5 years) have been conducted on dogs fed a control diet or a control diet + MH. This assessment included two cohorts of Labrador Retrievers. Cohort 1 included 39 neutered Labrador Retrievers, 12 male and 27 female, ranged in age from 5.1 to 8.2 years old at the beginning of the test-feeding period (mean age at the beginning of the study 6.7 years). Cohort 2 included 41 neutered Labrador Retrievers, 12 males and 29 females ranged in age from 2.0 to 6.1 years old at the beginning of the test feeding period (mean age at the beginning of the study 4.0 years). Each cohort was fed a control diet or a test diet where the only differences was inclusion of mHep-enriched avocado extract targeted to supply 2 mg/kg body weight of MH. All researchers, lab assistants, and assessors were blinded to the feeding groups.

Dogs were assessed during August and September each year. Each scorer was instructed to operate independently. Trained assessors were categorized as either familiar or unfamiliar to the dogs. The familiar assessors were people that worked with the dogs on a regular basis. Unfamiliar assessors did not interact with the dogs on a regular basis. Each assessing group was comprised of a handler and two scorers. All dogs were assessed with three people in the room with at least one familiar scorer in each group. This created a total of three possible conditions: familiar handler, two familiar scorers (Fam/Fam); familiar handler, one unfamiliar scorer, one familiar scorer (Fam/Unfam); unfamiliar handler, one unfamiliar scorer, one familiar scorer (Unfam/Unfam). The behaviors are outlined in Table 1.

*Calculating score:* Assessors were asked to score dogs on a scale from 1 - 5 including half scores. For the behavior items, a score of 1 signified "Fails To Do" while 5 signified "Excellent". For the demeanor items, a score of 1 signified "Strongly" while a score of 5 signified "Not at All". All necessary items were reverse scored to allow higher scores to indicate a more favorable score and lower score to indicate a less favorable score.

The sums of the corresponding behavior and demeanor items were used to calculate the score for each of the two behavior components. These scores were used to compare changes across years and between groups.

Table 1 - *Definition of Behavior Components with Corresponding Assessment Items*

| Social Interaction | Comfort in New Areas |
|---|---|
| Permits petting from standing handler inside kennel | Walks hallways & crosses thresholds minimal hesitation |
| Permits slip lead around neck | Enters testing room with minimal hesitation |
| Approaches standing handler in testing room | Approaches standing handler in testing room |
| Accepts petting in testing room | Accepts treats in testing room |
| Responds to play elicitation | Reaction to remote car (or rolling object) |
| Reaction to unknown dog-friendly dog | Shows aggression towards handler (growl, snarl, or bark aggressively) |
| Cringes away from handler | Tail tucked/submissive postures |
| Refuses or rejects treats | Refuses or rejects treats |
| Unwilling to interact/engage | Unwilling to interact/engage |
| Maximum possible mean = 5 | Maximum possible mean = 5 |

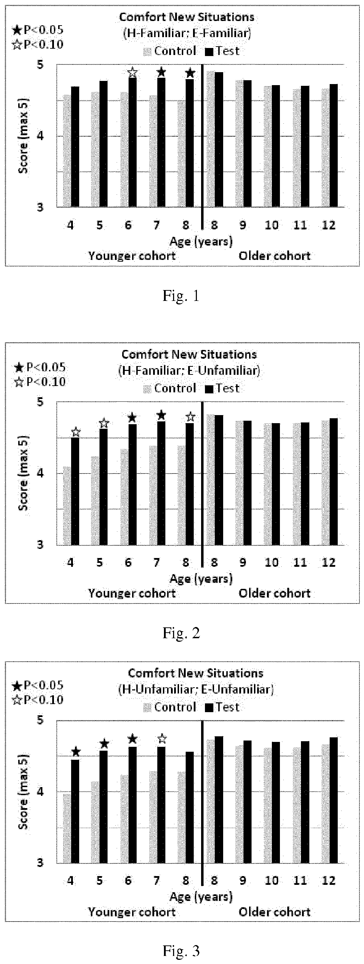

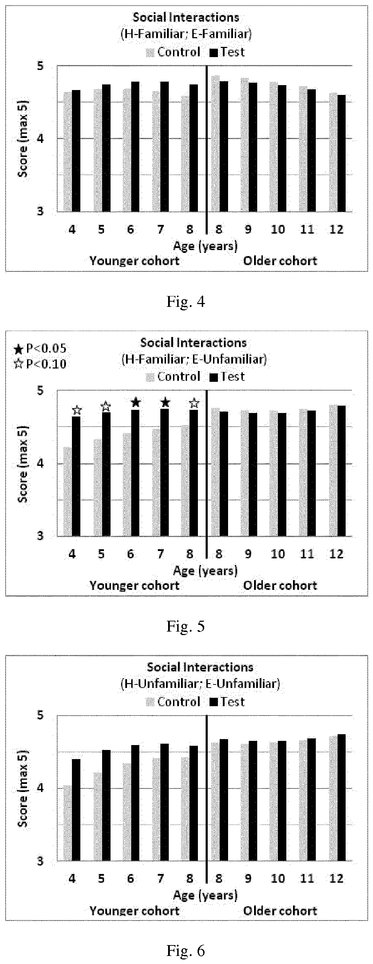

It was hypothesized that differences would occur due to the presence of an unfamiliar person and that 'comfort in new areas' would show a difference in later years and with unfamiliar testers and assessors. The results are presented in figures 1 - 6. The majority of differences for comfort in new areas occur in the expected condition (in the presence of an unfamiliar person) and at older ages. Specifically, the dogs in the familiar/familiar condition only started to show significant differences in later years. In addition, the differences in responding became significant between the two groups much earlier in the unfamiliar/unfamiliar condition with +MH dogs showing a significantly better response when challenged with introduction of a new environment.

When "social interaction" was assessed we hypothesized that dogs would interact more positively with people that are familiar. The ability to recognize individuals and retain that information is a complex cognitive task. Differences in this area of assessment occurred in the fam/unfam setting in later years indicating that the dogs were responding differently with the additional unfamiliar person in the room. In addition, the two groups were not different in the fam/fam or the unfam/unfam group. This may indicate that between the two diets, the group fed the supplement was able to focus on the familiar person present in the room. Without the difference in familiarity, the presence of the single unfamiliar person was enough to distract the control dogs versus the +MH group. +MH dogs were able to maintain focus on the familiar presence and continue to respond.

**Conclusion:** dogs fed with food comprising mannoheptulose had higher scores for ability to handle new areas and social interaction. Both these results suggest that mannoheptulose have a direct or indirect effect on arousal.

Example 2

Recently, the Applicant has investigated whether feeding MH would increase a cats' motivation to play, which could additionally improve the animal-human bond. The objectives of the study were to measure the influence of MH supplementation in cats and to measure the effects of dietary mannoheptulose (MH) treatment on the physiology and psychology of young adult, lean, and moderately overweight cats.

Study Design

Twenty cats (N=20) of similar age (~2.5 years), and split 10 female (5 lean and 5 moderately overweight) and 10 male (5 lean and 5 moderately overweight). To effectively test the effects of MH on energy metabolism dietary energy needs, intended to maintain weight, were provided equally between animals on a body weight basis; therefore, each cat received 45 kcal ME/kg body weight/d (females) and 50 kcal ME/kg body weight/d (males).

For two weeks prior to the initiation of the study cats were fed Iams® Original Chicken. Diets were presented in kibble form and cats were fed individually at 7:00 am each day and will be permitted 60 minutes to eat during food offerings. This feeding protocol were maintained throughout the entire study except on days in which fasted blood/calorimetry samples are obtained as cats were fed following the sampling period which was no later than 9:00 am. At the end of the first washout period cats were randomly allocated to either a control group or a control + MH group. On the first day of the study (Day 0) half the cats continued to receive the control diet without MH treatment (0 mg/kg body weight) and half of the cats were fed the control diet with MH treatment. Each cat was fed their respective diet for a total of 37d. For six days, after the first 37d dietary treatment, all cats were returned to the control diet without MH treatment that was used as the initial washout diet for the first part of the study for a second washout period. Following the washout period cats were fed the alternative diet for an additional 37d period. Therefore, this was a crossover study where all cats received both diets enabling each cat to act as its own control.

Behavioural Assessments to Measure Play Motivation:

Two walled stalls (each measuring: 100 cm W X 100 cm L X 75 cm H; Quenn City polymers, Dayton, Ohio), one classified as the start box and the other the goal box, each with a plexi-glass roof containing a 1 cm diameter hole in the center, were placed next to each other and connected via a swing door (23 cm W x 18 cm H). The swing door is made of 1/16" plexi-glass and is attached to the top of the door frame. The door is similar to the type cats are acclimated to use in their group living rooms. To assess play motivation, the swing door was made progressively more difficult to open through the addition of weights that were placed into a trough at the bottom of the door. When the cat pushes the weighted door with sufficient force it swings away from the frame, allowing the cat to pass underneath the door to enter the goal box where it was permitted to interact with the toy. Cats were assessed two times per dietary treatment on day 14 and again on day 37 at approximately 5 hrs post feeding. Cats were not permitted to interact with plush toys for four days prior to the initiation of the study. All procedures and measurements are adapted from Widowski and Duncan, 2000.

**Testing Procedure:** Each cat was individually removed from the group living room and placed in the start stall. A toy resembling a stuffed mouse was attached to a string and hung from the cut-out hole in the center of the plexi-glass roof of the goal stall only. After being released into the start stall, the cat was allowed 10 min to open the door to obtain access to the toy. Each testing series began with zero weight added to the door. If the cat successfully opened the door and entered the goal box, the cat was permitted 30 sec in contact with the toy. After 30 s, the cat was returned to the start box, and additional weights were added to the door. The procedure was repeated up to three times in a single test day. On the third trial of the day, cats were allowed to remain in the goal box for 2 min to provide sufficient time to play with the toy before returning to their group living room. If a cat did not attempt to open the door (after 10 minutes in the start stall), testing was finished for that day. On the second day of testing (d 37), the previous weight at which the cat had last successfully opened the door was used for trial 1 and then the weight was increased progressively with each trial. When a cat attempted to push the door open a total of 5 times but if unsuccessful, weights were removed, and the cat was returned to the start box to be tested again with a reduced weights to define the threshold of work to obtain a toy. In addition to obtaining the maximum door weights that each cat would push to enter the goal box, other behaviour patterns were measured to assess motivation. The latency to open the door, number of unsuccessful attempts to open the door, and the latencies from opening the door to first intentional contact with the toy were measured. Results: Cats fed the test diet containing the MH spent less time in the start box, had more successful trials, had fewer non-successful trials, and pushed more weight to obtain the toy than did cats fed the control diet (Table 2).

Table 2: Motivational measurements of play in cats

| Behavior | Test | Control | P-Value |
|---|---|---|---|
| Mean duration (s) in start box on max weight | 100.8 $\pm$ 25* | 185.39 $\pm$ 26** | 0.02 |
| Mean number of successful trials | 5.15 $\pm$ 0.5** | 2.90 $\pm$ 0.6* | 0.006 |
| Mean number of non-successful trials | 0.25 $\pm$ 0.1* | 0.68 $\pm$ 0.1** | 0.007 |
| Mean Maximum Weight (g) | 407.5 $\pm$ 46.1** | 227.8 $\pm$ 47.4 | 0.003 |

**Conclusions:** These data strongly suggest that cats fed MH have significantly greater motivation to play than cats being fed the same diets, but without MH.

Example 4

Brain serotonin is known to influence the mood. Serotonin is synthesized in the brain from tryptophan, which uptake into the brain is dependent on the plasma ratio of tryptophan to the sum of other large neutral amino acids (Trp/LNAA). Other large amino acids include leucine, isoleucine, valine, phenylalanmine and tyrosine. It is well accepted that carbohydrate rich diets increase this ration and protein rich diets decrease the ration, in other words, when there is decrease in insulin sensitivity and a greater glycemic response, greater Trp/LNAA ratios are expected.

Blood samples for amino acid analysis were collected from a total of 51 dogs fed a control diet or a control diet + MH. The blood samples were collected from two cohorts of Labrador Retrievers. Cohort 1 included 19 neutered Labrador Retrievers, 5 male and 14 female, ranged in age from 12.6 to 15.7 years old at the time of sampling. Cohort 2 included 32 neutered Labrador Retrievers, 9 males and 23 females ranged in age from 8.3 to 12.5 years old at the time of sampling. Each cohort was fed similar a control diet or a test diet where the only differences was inclusion of mHep enriched avocado juice concentrate targeted to supply 200 ppm MH in the diet. The avocado juice concentrate was derived from whole fruit (flesh, peel and pit) avocados. Cohort 1 dogs had been consuming the control or test diets continuously for 90 months at the time of sampling. Cohort 2 dogs had been consuming the control or test diets continuously for 77 months at the time of sampling. All researchers, lab assistants, and assessors were blinded to the feeding groups.

**Plasma/serum sample prep for HPLC analysis**

**Deproteinating the plasma/serum sample**

1. Mix 100 $\mu$L of thawed plasma or serum with 100 $\mu$L of 0.4 mM norleucine solution into a labeled 10K spin filter centrifuge tube

2. Centrifuge at 15,000 x g for 30 minutes at 4 °C.

3. Remove from the centrifuge, discard the insert and re-close the centrifuge tube that contains the flow through (deproteinated sample). At this point, the deproteinated sample may be stored at -20°C until further processing, or you may proceed to the following step.

4. Take a 50 $\mu$L aliquot of the deproteinated sample and transfer it to a labeled Kimble glass tube and place in a freeze drier flask and on the freeze drier until they are dry.

5. Cover the dried Kimble tubes with parafilm and store at -20°C until the time of processing.

**Re-dry step**

1. Remove samples from the freezer and remove the parafilm. At the same time, remove 2 predried "complete AA standard" Kimble tubes (see "making amino acid standards" for directions).
2. To the "complete AA standard" add 25 $\mu$L of 0.2 mM glutamine
3. To each sample/standard vial add 10 $\mu$L of re-dry solution and vortex the tubes. Make the re-dry solution in the fume hood (the TEA is smelly!)

| Compound | Parts | 10 vials | 20 vials | 30 vials | 40 vials | 50 vials | 60 vials |
|---|---|---|---|---|---|---|---|
| 1 M Na Acetate | 2 | 60 $\mu$L | 120 $\mu$L | 180 $\mu$L | 240 $\mu$L | 300 $\mu$L | 360 $\mu$L |
| Triethylamine (TEA) | 1 | 30 $\mu$L | 60 $\mu$L | 90 $\mu$L | 120 $\mu$L | 150 $\mu$L | 180 $\mu$L |
| Methanol | 2 | 60 $\mu$L | 120 $\mu$L | 180 $\mu$L | 240 $\mu$L | 300 $\mu$L | 360$\mu$L |

4. Dry samples on the freezer drier for at least 30 minutes.

**Derivatizing step**

1. Remove samples from the freeze drier.

2. To each vial, add 20 µL of derivatizing solution and vortex the tubes. Make the derivatizing solution in the fumehood.

| Compound | Parts | 10 vials | 20 vials | 30 vials | 40 vials | 50 vials | 60 vials |
|---|---|---|---|---|---|---|---|
| ddH2O | 1 | 25 µL | 50 µL | 75 µL | 100 µL | 125 µL | 150 µL |
| Triethylamine | 1 | 25 µL | 50 µL | 75 µL | 100 µL | 125 µL | 150 µL |
| PITC | 1 | 25 µL | 50 µL | 75 µL | 100 µL | 125 µL | 150 µL |
| Methanol | 7 | 175 µL | 350 µL | 525 µL | 700 µL | 875 µL | 1050 µL |

3. Put samples into the freeze dry flask to incubate at room temperature for 20 minutes
4. Place the flask on the freeze drier until dry (at least 45 minutes).

Re-diluting samples for HPLC

1. Add 100 µL of Physiol A buffer to each standard and sample, vortex well.

2. Using a glass Pasteur pipette, transfer all contents in the Kimble tube into a small glass insert tube that will fit inside of the larger autosampler vials- pay close attention to which number of vial you are putting each sample into and make sure the vial number corresponds to what is specified in your sample line-up on the computer. Red septa go on the standard vials and white septa go on the sample vials.

3. Place the autosampler vial carousel into the HPLC system.

Measure the samples with HPLC.

**Buffer making**

*Physiol A*

**Generally, 2 or 4 L of Physiol A are made at a time.

1. Measure 2 (or 4) L of nanopure water and put into a large plastic beaker

2. Remove 700µL/2L (1400µL/4L) of water from the beaker and discard

3. Add 700 µL/2L (1400 µL/4L) of 10 mg/mL EDTA disodium dihydrate to the beaker

4. Weigh out 19.05 g/2L (38.10 g/2L) of sodium acetate and add to beaker

5. Mix the solution on a stir plate until all the sodium acetate is dissolved.

6. pH the solution and add glacial acetic acid dropwise to bring the pH down to 6.45 ($\pm$ 0.01)

7. Filter the solution to remove any air

8. Measure out 1950 mL/2L (3900 mL/4L) of the filtered solution and then add 50 mL/2 L (100 mL/4 L) of acetonitrile (note: measure these out separately, do not just add the acetonitrile to the top of the graduated cylinder as this will not work).

9. Carefully pour this new solution into the appropriate glass bottle attached to the HPLC (labeled Physiol A) and avoid introducing any bubbles.

*Physiol B:*

**I usually make 1 L of this at a time

1. Mix the following together in a beaker:

a. 450 mL acetonitrile
b. 400 mL nanopure water
c. 150 mL methanol

2. Filter the solution to remove any air.
3. Carefully pour this solution into the appropriate glass bottle attached to the HPLC (labeled Physiol B) and avoid introducing any bubbles.

**Making amino acid standards**

*Stock solutions for extra amino acids:*

1. Make stock solutions (~100 μmol/mL = 0.100 mmol/mL = 100 mmol/L) for each of:

- L-asparagine (MW = 132.12 mg/mmol)- weigh out ~ 26 mg
- L-citrulline (MW = 175.19 mg/mmol)- weigh out ~ 36 mg
- L-cysteine hydrochloride (MW = 175.16 mg/mmol)- weigh out ~ 36 mg
- L-glutamine (MW = 146.15 mg/mmol)- weigh out ~ 30 mg
- L-norleucine (MW = 131.17 mg/mmol)- weigh out ~ 26 mg
- L-ornithine hydrochloride (MW = 165.62 mg/mmol)- weigh out ~ 34 mg

2. For each amino acid, weigh the amino acid directly into a 2 mL centrifuge tube and record the exact weight (to 0.1 mg). Then, add exactly 2 mL of nanopure water to the centrifuge tube, close the tube and vortex to dissolve all powder.
3. Calculate the exact concentration of each amino acid and record that on the centrifuge tube:

$$Concentration\ (in\ mmol/mL) = [\ (weight\ in\ mg)/(MW\ in\ mg/mmol)]/2\ mL$$

4. Store at -20 °C until use

*0.4 mM norleucine solution (internal standard):*

1. Make 50 mL (= 50000 μL) of solution at a time in a 50 mL volumetric flask
2. From the norleucine stock solution concentration, calculate the amount needed using:

$$Amount\ (in\ μL) = [(50000\ μL)*(0.4\ mmol/L)]/(stock\ solution\ conc.)$$

3. Vortex the stock solution and pipet the desired amount of stock solution into the volumetric flask
4. Bring the volume in the volumetric flask up to (exactly) 50 mL using 0.1 N HCl.
*0.1N HCl is made by adding 4.1 mL of ~12.2 N HCl stock [stock is ~37.5% HCl (v/v), with a MW of 36.46 g/mol and a density of 1.19 g/mL] to ~250 mL nanopure water in a volumetric flask and then bringing the volume up to 500 mL with nanopure water.
5. Transfer into an appropriate storage container
6. Store at 4 °C until use

*0.2 mM glutamine solution (added to the complete AA standard during the re-dry step):*

1. Make 50 mL of solution at a time in a 50 mL volumetric flask
2. From the glutamine stock solution concentration, calculate the amount needed using:

$$Amount\ (in\ \mu L) = [(50000\ \mu L)*(0.2\ mmol/L)]/(stock\ solution\ conc.)$$

3. Vortex the stock solution and pipet the desired amount of stock solution into the volumetric flask
4. Bring the volume in the volumetric flask up to (*exactly*) 50 mL using nanopure water
5. Transfer into an appropriate storage container
6. Store at 4 °C until use

*0.5 mM extra AA standard solution:*

1. Make 50 mL of solution at a time in a 50 mL volumetric flask
2. For each of asparagine, cysteine hydrochloride, ornithine hydrochloride and citrulline, calculate the amount of each stock solution needed using:

$$Amount\ (in\ \mu L) = [(50000\ \mu L)*(0.5\ mmol/L)]/(stock\ solution\ conc)$$

3. Vortex each stock solution tube and pipet the desired amount of stock solution into the volumetric flask
4. Once all amino acid stock solutions have been added, bring the volume in the volumetric flask up to (*exactly*) 50 mL using nanopure water
5. Transfer into an appropriate storage container
6. Store at 4 °C until use

*Making the complete AA standard (0.2 mM = 200 nmol/mL of each AA):*

1. Mix 500 $\mu$L of the pre-made standard (Sigma product A2908; 0.5 mM) and 500 $\mu$L of the extra AA standard (0.5 mM) and 250 $\mu$L of nanopure water in a centrifuge tube.
2. Vortex to mix
3. Put 25 $\mu$L aliquots (5 nmol) into individual labeled Kimble glass tubes. Place in a freeze drier flask and on the freeze drier until they are dry.

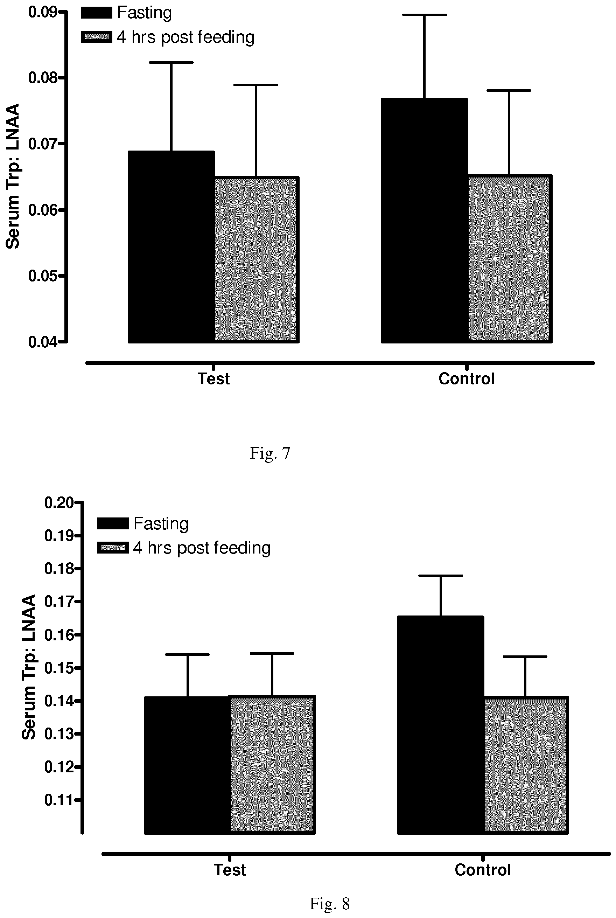

Figure 7 is an illustration of old and young cohorts pooled together, the time by diet interaction showed a statistical trend (P= 0.15). Dogs fed mannoheptulose had no differences in fasting and 4 hrs post fed Trp: LNAA concentrations, whereas there was a significant (P<0.05) decrease in the Trp: LNAA concentrations in dogs fed the control diet.

Figure 8 is an illustration of the situation when the cohorts were examined separately, the time by diet interaction (P= 0.0006) and time (P=0.0008) was highly significant for Cohort 1 where the dogs are older as compared to cohort 2. Dogs fed diets containing MH had no differences (P>0.05) in fasting and 4 hrs post fed Trp: LNAA concentrations, whereas there was a significant (P<0.05) decrease in the Trp: LNAA concentrations in older dogs fed the control diet. The differences in ratio were not driven by differences in Tryptophan or the LNAA alone, as evidenced by a lack of statistical difference between dietary treatments or fasting vs. feeding.

There were no differences in the Trp: LNAA ratio (P>0.05) in younger dogs fed the test or control diets (Cohort 2) or between fasting and feeding, although there was a significant increase (P<0.05) in the concentration of LNAA (Leucine, Isoleucine, Valine, Phenylalanine and Tyrosine) from fasting to feeding in.

Table 3 illustrates two kibble compositions having the following components at the approximate indicated amounts are prepared using methods which are standard in the art, including extrusion, and are fed to dogs and/or cats as a daily feed:

Table 3

| Component | Example 4A (Component Amount indicated as Wt% ) | Example 4B (Component Amount indicated as Wt%) | Example 4C (Component Amount indicated as Wt%) |
|---|---|---|---|
| Extract of Avocado* | 0.02 | 0.01 | 0.08 |
| Chicken, Chicken By-product Meal, Fish Meal, and Egg | 44 | 47 | 44 |
| Chicken Fat | 8 | 6 | 6.5 |
| Beet Pulp | 2 | 3 | 3 |
| Salts | 2.5 | 2 | 0.8 |
| Vitamins and Minerals** | 1 | 1 | 1.2 |
| Minors*** | 3.5 | 4 | 2 |
| Grains | Remainder | Remainder | Remainder |

*Criollo Avocado may be substituted with other plant matter having enhanced mannoheptulose content. The incorporation of a mannoheptulose source likely replaces a similar amount of a grain source in the composition. The extract comprises about 10% of Mannoheptulose.

**Vitamins and Minerals may include: Vitamin E, beta-carotene, Vitamin A, Ascorbic Acid, Calcium Pantothenate, Biotin, Vitamin $B_{12}$, Vitamin $B_1$, Niacin, Vitamin $B_2$, Vitamin $B_6$, Vitamin $D_3$, Vitamin $D_2$, Folic Acid, Choline Chloride, Inositol, Calcium Carbonate, Dicalcium Phosphate, Potassium Chloride, Sodium Chloride, Zinc Oxide, Manganese Sulfate, Copper Sulfate, Manganous Oxide, Ferrous Sulfate, Potassium Iodide, Cobalt Carbonate.

***Minors may include: Fish oil, flax seed, flax meal, cellulose, flavors, antioxidants, taurine, yeast, carnitine, chondroitin sulfate, glucosamine, lutein, rosemary extract.

Example 5

Table 4 illustrates beef-flavor gravy composition is prepared by combining the following components in a conventional manner:

Table 4

| Component | Wt% |
|---|---|
| Mannoheptulose* | 0.14 |
| Chicken Fat | 3.0 |
| Spray-Dried Beef Particles and Broth | 3.0 |
| Xanthan Gum | 0.5 |
| Flax Seed | 0.2 |
| Vegetables | 0.2 |
| Vitamins** | 0.06 |
| Minerals** | 0.04 |
| Phosphoric Acid | 0.95 |
| Beef Flavor | 0.1 |

(continued)

| Component | Wt% |
|---|---|
| Water | balance |

*Mannoheptulose may be substituted with another glucose anti-metabolite.
**Vitamins and Minerals may include: Vitamin E, beta-carotene, Vitamin A, Ascorbic Acid, Calcium Pantothenate, Biotin, Vitamin $B_{12}$, Vitamin $B_1$, Niacin, Vitamin $B_2$, Vitamin $B_6$, Vitamin $D_3$, Vitamin $D_2$, Folic Acid, Choline Chloride, Inositol, Calcium Carbonate, Dicalcium Phosphate, Potassium Chloride, Sodium Chloride, Zinc Oxide, Manganese Sulfate, Copper Sulfate, Manganous Oxide, Ferrous Sulfate, Potassium Iodide, Cobalt Carbonate.

One fluid ounce of the gravy composition is mixed with one-half cup of a food composition daily prior to feeding to a mammal. Amounts of the gravy composition are determined as desired by the guardian of the mammal.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. Use of nutritionally balanced food or pet food composition comprising a glucose anti-metabolite and/or source of glucose anti-metabolite in a method of enhancing a positive affect and/or energetic arousal in a mammal, wherein said glucose anti-metabolite is selected from the group consisting of 2-deoxy-D-glucose; 5-thio-D-glucose; 3-O-methylglucose; 1,5-anhydro-D-glucitol; 2,5-anhydro-D-mannitol; mannoheptulose; and mixtures thereof, and wherein said enhancing a positive affect and/or energetic arousal in said mammal is selected from the group consisting of enhanced social interaction, enhanced obedience, enhanced manners, enhanced awareness, enhanced activity and mixtures thereof.

2. Use according to claim 1, wherein said glucose anti-metabolite is mannoheptulose.

3. Use according to claim 2, wherein said mannoheptulose is from the plant material selected from the group consisting of an avocado extract, avocado meal, alfalfa, fig and primrose and mixtures hereof.

4. Use according to any of the preceding claims, wherein said composition comprises from 0.0001 to 10% by weight of the composition of said glucose anti-metabolite, preferably from 0.001 to 5%, more preferably from 0.001 to 1.5 and most preferably from 0.01 to 0.5%.

5. Use according to any of the preceding claims, wherein dosage of said glucose anti-metabolite to mammal on daily basis is from 0.5 to 200 mg/kg, wherein mg is the level of glucose anti-metabolite and kg is kilogram of bodyweight of the mammal, preferably from 1 to 150 mg/kg, more preferably from 2 to 100 mg/kg and most preferably from 2 to 50mg/kg.

6. Use according to claim 5, wherein said glucose anti-metabolite is mannoheptulose, dosage to mammal on daily basis is from 0.5 to 20 mg/kg, wherein mg is the level of glucose anti-metabolite and kg is kilogram of bodyweight of the mammal, preferably from 1 to 10 mg/kg, more preferably from 2 to 8mg/kg.

7. The use according to any of the preceding claims, wherein said composition is selected from the group consisting of nutritionally balanced dog food and nutritionally balanced cat food.

8. The use according to any of the preceding claims, wherein said composition is a food supplement selected from the group consisting of, treats, chew, biscuits, gravy, sauce, beverage, supplemental water, yogurt, powder and combinations thereof.

9. The use according to any preceding claims, wherein said composition further comprises animal protein, plant protein, farinaceous matter, vegetables, fruit, egg-based materials, undenatured proteins, food grade polymeric adhesives, gels, polyols, starches, gums, flavorants, seasonings, salts, colorants, time-release compounds, minerals, vitamins, antioxidants, prebiotics, probiotics, aroma modifiers, lipids, and combinations thereof.

**Patentansprüche**

1. Verwendung einer ernährungsphysiologisch ausgewogenen Nahrungsmittel- oder Haustierfutterzusammensetzung, die einen Glucoseantimetaboliten und/oder eine Glucoseantimetabolitenquelle umfasst, in einem Verfahren zur Verbesserung eines positiven Affekts und/oder einer energetischen Aktivierung bei einem Säugetier, wobei der Glucoseantimetabolit aus der Gruppe ausgewählt ist, die aus 2-Deoxy-D-Glucose; 5-thio-D-Glucose; 3-O-Methylglucose; 1,5-Anhydro-D-Glucitol; 2,5-Anhydro-D-Mannitol; Mannoheptulose; und Gemischen aus diesen besteht, und wobei die Verbesserung eines positiven Affekts und/oder einer energetischen Aktivierung bei einem Säugetier aus der Gruppe ausgewählt ist, die aus verbesserter sozialer Interaktion, verbesserter Gehorsamkeit, verbesserten Manieren, verbesserter Aufmerksamkeit, verbesserter Aktivität und Mischungen von diesen besteht.

2. Verwendung nach Anspruch 1, wobei es sich bei dem Glucoseantimetaboliten um Mannoheptulose handelt.

3. Verwendung nach Anspruch 2, wobei die Mannoheptulose aus einem Pflanzenmaterial stammt, das aus der Gruppe ausgewählt ist, die aus Avocadoextrakt, Avocadomehl, Alfalfa, Feige und Primel und Gemischen aus diesen besteht.

4. Verwendung nach einem der vorhergehenden Ansprüche, wobei die Zusammensetzung 0,0001 bis 10 %, in Gewichtsprozent der Zusammensetzung, an Glucoseantimetabolit, vorzugsweise 0,001 bis 5 %, bevorzugter 0,001 bis 1,5 und am bevorzugtesten 0,01 bis 0,5 % umfasst.

5. Verwendung nach einem der vorhergehenden Ansprüche, wobei die Dosierung des Glucoseantimetaboliten für das Säugetier auf einer täglichen Basis 0,5 bis 200 mg/kg, wobei es sich bei mg um die Menge des Glucoseantimetaboliten und bei kg um Kilogramm Körpergewicht des Säugetiers handelt, vorzugsweise 1 bis 150 mg/kg, bevorzugter 2 bis 100 mg/kg und am bevorzugtesten 2 bis 50 mg/kg beträgt.

6. Verwendung nach Anspruch 5, wobei es sich bei dem Glucoseantimetaboliten um Mannoheptulose handelt, die Dosierung für das Säugetier auf einer täglichen Basis 0,5 bis 20 mg/kg, wobei es sich bei mg um die Menge des Glucoseantimetaboliten und bei kg um Kilogramm Körpergewicht des Säugetiers handelt, vorzugsweise 1 bis 10 mg/kg, bevorzugter 2 bis 8 mg/kg beträgt.

7. Verwendung nach einem der vorhergehenden Ansprüche, wobei die Zusammensetzung aus der Gruppe ausgewählt ist, die aus ernährungsphysiologisch ausgewogenem Hundefutter und ernährungsphysiologisch ausgewogenem Katzenfutter besteht.

8. Verwendung nach einem der vorhergehenden Ansprüche, wobei es sich bei der Zusammensetzung um ein Nahrungsergänzungsmittel handelt, das aus der Gruppe ausgewählt ist, die aus Leckerlis, Kausnacks, Keksen, Bratensaft, Sauce, Getränken, ergänzendem Wasser, Joghurt, Pulver und Mischungen von diesen besteht.

9. Verwendung nach einem der vorhergehenden Ansprüche, wobei die Zusammensetzung darüber hinaus tierisches Protein, pflanzliches Protein, mehlhaltigen Stoff, Gemüse, Obst, Materialien auf Eierbasis, undenaturierte Proteine, polymere Klebstoffe in Nahrungsmittelqualität, Gels, Polyole, Stärken, Gummis, Aromastoffe, Gewürze, Salze, Farbstoffe, zeitlich sich freisetzende Verbindungen, Mineralien, Vitamine, Antioxidantien, Präbiotika, Probiotika, Aromamodifikatoren, Lipide und Kombinationen von diesen umfasst.

**Revendications**

1. Utilisation d'une composition alimentaire ou d'une composition alimentaire pour animal domestique nutritionnellement équilibrée comprenant un antimétabolite de glucose et/ou une source d'antimétabolite de glucose dans un procédé d'amélioration d'un affect positif et/ou d'une activation énergétique chez un mammifère, sachant que ledit antimétabolite de glucose est sélectionné dans le groupe constitué par le 2-déoxy-D-glucose ; le 5-thio-D-glucose ; le 3-O-méthylglucose ; le 1,5-anhydro-D-glucitol ; le 2,5-anhydro-D-mannitol ; le mannoheptulose ; et leurs mélanges, et sachant que ladite amélioration d'un affect positif et/ou d'une activation énergétique chez ledit mammifère est sélectionnée dans le groupe constitué par une interaction sociale améliorée, une obéissance améliorée, des manières améliorées, une prise de conscience améliorée, une activité améliorée et leurs mélanges.

**2.** Utilisation selon la revendication 1, sachant que ledit antimétabolite de glucose est du mannoheptulose.

**3.** Utilisation selon la revendication 2, sachant que ledit mannoheptulose est issu de la matière végétale sélectionnée dans le groupe constitué par un extrait d'avocat, la farine d'avocat, la luzerne, la figue, la primevère et leurs mélanges.

**4.** Utilisation selon l'une quelconque des revendications précédentes, sachant que ladite composition comprend de 0,0001 à 10 % en poids de la composition dudit antimétabolite de glucose, de préférence de 0,001 à 5 %, de façon plus préférentielle de 0,001 à 1,5 et de façon la plus préférentielle de 0,01 à 0,5 %.

**5.** Utilisation selon l'une quelconque des revendications précédentes, sachant que la dose dudit antimétabolite de glucose à administrer quotidiennement au mammifère est de 0,5 à 200 mg/kg, sachant que mg correspond au niveau d'antimétabolite de glucose et kg correspond au poids corporel du mammifère en kilogrammes, de préférence de 1 à 150 mg/kg, de façon plus préférentielle de 2 à 100 mg/kg et de façon la plus préférentielle de 2 à 50 mg/kg.

**6.** Utilisation selon la revendication 5, sachant que ledit antimétabolite de glucose est du mannoheptulose, la dose à administrer quotidiennement au mammifère est de 0,5 à 20 mg/kg, sachant que mg correspond au niveau d'antimétabolite de glucose et kg correspond au poids corporel du mammifère en kilogrammes, de préférence de 1 à 10 mg/kg, de façon plus préférentielle de 2 à 8 mg/kg.

**7.** L'utilisation selon l'une quelconque des revendications précédentes, sachant que ladite composition est sélectionnée dans le groupe constitué par un aliment pour chien nutritionnellement équilibré et un aliment pour chat nutritionnellement équilibré.

**8.** L'utilisation selon l'une quelconque des revendications précédentes, sachant que ladite composition est un complément alimentaire sélectionné dans le groupe constitué par les friandises, les chiques, les biscuits, les jus, les sauces, les boissons, les eaux de complément, les yaourts, les poudres et leurs combinaisons.

**9.** L'utilisation selon l'une quelconque des revendications précédentes, sachant que ladite composition comprend en outre de la protéine animale, de la protéine végétale, de la matière farineuse, des légumes, des fruits, des matières à base d'oeuf, des protéines non dénaturées, des colles polymères alimentaires, des gels, des polyols, des amidons, des gommes, des essences, des condiments, des sels, des colorants, des composés à libération retardée, des minéraux, des vitamines, des antioxydants, des prébiotiques, des probiotiques, des modificateurs d'arôme, des lipides, et leurs combinaisons.

Fig. 1

Fig. 2

Fig. 3

Fig. 4

Fig. 5

Fig. 6

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2002035071 A
- US 20020035701 A
- US 20050158294 A

### Non-patent literature cited in the description

- **ROWLAND et al.** *Physiology and Behavior,* May 1983, vol. 30, 5
- **LANE et al.** *J. Anti-Aging Med.,* 1998, vol. 1 (4), 327-337
- **HOFFMAN et al.** *Biochemistry,* 1968, vol. 7, 4479-4483
- **CHEN et al.** *Arch. Biochem. Biophys.,* 1975, vol. 169, 392-396
- **JAY et al.** *J. Neurochem.,* 1990, vol. 55, 989-1000
- **SOLS et al.** *J. Biol. Chem.,* 1954, vol. 210, 581-595
- **CRANE et al.** *J. Biol. Chem.,* 1954, vol. 210, 597-696
- **RIQUELME et al.** *Proc. Natl. Acad. Sci. USA,* 1983, vol. 80, 431-435
- **SIMON et al.** *Arch. Biochem. Biophys,* 1957, vol. 69, 592-601